(19) Europäisches Patentamt
European Patent Office
Office européen des brevets

(11) Veröffentlichungsnummer: **0 033 775**
**B1**

(12) **EUROPÄISCHE PATENTSCHRIFT**

(45) Veröffentlichungstag der Patentschrift: **17.11.82**

(51) Int. Cl.³: **C 07 C 139/00**, C 07 C 139/14

(21) Anmeldenummer: **80107658.9**

(22) Anmeldetag: **05.12.80**

(54) **Verfahren zur Abtrennung von Sulfonsäuren aus dem bei der Umsetzung von Paraffinen mit Schwefeldioxid, Sauerstoff und Wasser in Gegenwart von UV-Licht erhaltenem Reaktionsprodukt.**

(30) Priorität: **08.02.80 DE 3004651**

(43) Veröffentlichungstag der Anmeldung: **19.08.81 Patentblatt 81/33**

(45) Bekanntmachung des Hinweises auf die Patenterteilung: **17.11.82 Patentblatt 82/46**

(84) Benannte Vertragsstaaten: **BE FR GB IT NL**

(56) Entgegenhaltungen:
**US-A-4 177 208**
**D'ANS LAX: »Taschenbuch für Chemiker und Physiker«, dritte neu bearb. Aufl., Band II, 1964, Springer-Verlag Berlin, DE. * Seite 193 ***

(73) Patentinhaber: **CHEMISCHE WERKE HÜLS AG, Postfach 1320, D-4370 Marl 1 (DE)**

(72) Erfinder: **Springmann, Hermann, Dr., Am Mühlenberg 2, D-4358 Haltern (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 033 775 B1

BUNDESDRUCKEREI BERLIN

Verfahren zur Abtrennung von Sulfonsäuren aus dem bei der Umsetzung von Paraffinen mit Schwefeldioxid, Sauerstoff und Wasser in Gegenwart von UV-Licht erhaltenem Reaktionsprodukt

Es ist bekannt, daß aliphatische und cycloaliphatische Alkohole mit mindestens 5 Kohlenstoffatomen im Molekül als Extraktionsmittel eingesetzt werden können, um aus einem rohen Sulfonierungsgemisch, das neben den erwünschten Sulfonsäuren noch nicht umgesetztes Paraffin, Wasser und Schwefelsäure enthält, die organischen Bestandteile zu extrahieren (DE-OS 2 139 477). Die Schwefelsäure scheidet sich dabei in der wäßrigen Phase ab und kann somit von dem alkoholischen Extrakt abgetrennt werden. Das vorhandene, nicht umgesetzte Paraffin bleibt mit den Sulfonsäuren zusammen in der leichteren organischen Phase. Die gesamte vorhandene Paraffinmenge wird dann nach Neutralisation der Sulfonsäuren und Entfernung des Extraktionsmittels in einer weiteren Verfahrensstufe von den als Alkalisulfonaten vorliegenden Sulfonsäuren abgetrennt.

Aus der DE-PS 827 065 ist es weiterhin bekannt, Alkalisulfonate hochmolekularer aliphatischer Kohlenwasserstoffe dadurch zu reinigen und konzentrieren, daß ihre wäßrigen Lösungen, wie sie durch Versetzen der technischen Verseifungsprodukte von entsprechenden höhermolekularen aliphatischen Kohlenwasserstoffsulfochloriden mit Mineralsäure erhalten werden, mit höheren, in Wasser nicht oder sehr wenig löslichen Alkoholen bzw. deren Gemischen extrahiert werden und die Alkohollösung anschließend nacheinander mit Wasser und Alkalilauge behandelt wird.

Für die Extraktion kommen höhere aliphatische oder aromatische Alkohole in Frage, vorzugsweise hydroaromatische Alkohole, wie z. B. Cyclohexanol und Methylcyclohexanol. Im Gegensatz zum Verfahren der DE-OS 2 139 477 werden bei dem Verfahren der DE-PS 827 065 das nicht umgesetzte Paraffin in einer separaten Verfahrensstufe bereits vor der Behandlung mit dem Alkohol abgetrennt.

Gemäß beiden Verfahren des Standes der Technik wird somit das nicht umgesetzte Paraffin und die als Nebenprodukt anfallende Schwefelsäure (im Falle der Sulfoxidation) bzw. anfallenden Chloride (im Falle der Sulfochlorierung) in zwei separaten Verfahrensschritten aus dem Sulfonierungsgemisch entfernt.

Aufgabe der vorliegenden Erfindung war es daher, ein Verfahren zu entwickeln, das es gestattet, aus dem bei der Umsetzung von Paraffinen mit Schwefeldioxid, Sauerstoff und Wasser in Gegenwart von ultraviolettem Licht erhaltenen Reaktionsprodukt mit einem Verfahrensschritt sowohl die Schwefelsäure als auch das nicht umgesetzte Paraffin weitgehend von den gewünschten Sulfonsäuren abzutrennen.

Diese Aufgabe wurde überraschenderweise durch die in den Patentansprüchen beschriebene Maßnahme gelöst.

Das erfindungsgemäße Verfahren geht demnach in erster Linie von dem rohen Reaktionsprodukt aus, das bei der bekannten Umsetzung von Paraffinen mit Schwefeldioxid, Sauerstoff und Wasser in Gegenwart von ultraviolettem Licht erhalten wird (Sulfoxidation nach dem Licht/Wasser-Verfahren; siehe Chemie in unserer Zeit, 13 [1979], Seite 157 ff.). Daneben können natürlich auch nach anderen Verfahren erhaltene Sulfonierungsgemische eingesetzt werden, sofern sie mit den erfindungsgemäß einsetzbaren Alkoholen im Sinne der Aufgabenstellung ein dreiphasiges System bilden.

Zur Abtrennung der Sulfonsäuren geeignete Alkohole der allgemeinen Formel

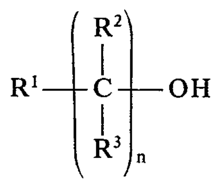

in der $R^1$ einen gegebenenfalls durch Halogenatome und/oder Alkylgruppen mit 1 bis 6 Kohlenstoffatomen, vorzugsweise 1 bis 3 Kohlenstoffatomen, substituierten Phenylrest darstellt, $R^2$ und $R^3$ Wasserstoff bedeuten, n eine ganze Zahl von 1 bis 3 ist und $R^2$ auch eine Methylgruppe sein kann, wenn n gleich 1 ist, sind z. B. Benzylalkohol, $\alpha$-Phenylethanol, $\beta$-Phenylethanol, 3-Phenylpropanol-(1) und p-Methylbenzylalkohol, wobei der Benzylalkohol bevorzugt ist.

Im allgemeinen geht man bei der Durchführung des erfindungsgemäßen Verfahrens so vor, daß man den Sulfoxidationsaustrag bei einer Temperatur von 10 bis 60° C, vorzugsweise bei 20 bis 40° C, mit 20 bis 100 Gewichtsprozent, vorzugsweise 40 bis 60 Gewichtsprozent, besonders bevorzugt mit der ca. halben Gewichtsmenge (50 Gewichtsprozent) des aromatischen Alkohols versetzt und gut durchmischt. Die Durchmischung kann in jedem handelsüblichen Mischaggregat, wie z. B. einem Rührkessel, durchgeführt, die optimale Mischzeit leicht durch einige orientierende Versuche ermittelt werden. Nach einer kurzen Absitzzeit (ca. 1 bis 2 Minuten) bilden sich drei klare Trennschichten aus, von denen die obere weitgehend aus Paraffin besteht, die mittlere die Sulfonsäuren in dem Extraktionsmittel gelöst enthält und in der unteren Schicht die Schwefelsäure als im allgemeinen 15- bis 25gewichtsprozentige, vorzugsweise 18- bis 21gewichtsprozentige, wäßrige Lösung vorliegt. Die drei Schichten lassen sich leicht voneinander trennen. Die Trennung kann sowohl kontinuierlich als auch diskontinuierlich durchgeführt werden.

Die Aufarbeitung der alkoholischen (mittleren) Phase kann auf verschiedene Art und Weise erfolgen. Sie richtet sich in erster Linie danach, ob man die freien Sulfonsäuren oder die Sulfonate, vorzugsweise Alkalisulfonate, erhalten will.

Um die freien Sulfonsäuren zu erhalten, entfernt man den aromatischen Alkohol z. B. durch azeotrope Destillation bei einem Druck, der höchstens gleich dem Atmosphärendruck ist. Zu diesem Zweck steuert man die Wassermenge in der alkoholischen Lösung der Sulfonsäuren so, daß die Gesamtmenge des Alkohols im azeotropen Gemisch mit dem Wasser abdestilliert werden kann. Die Sulfonsäuren werden im Bodenteil der Kolonne in Form einer Flüssigkeit gewonnen, die gegebenenfalls zur Überführung in Sulfonate neutralisiert werden kann.

Zur Abtrennung der Sulfonsäuren in Form von Sulfonaten geht man so vor, daß man den alkoholischen Extrakt vor der Abtrennung des Alkohols mit einer basischen Verbindung behandelt. Geeignete basische Verbindungen sind Hydroxide und Carbonate der Alkalimetalle, vorzugsweise des Natriums und Kaliums, sowie Oxide, Hydroxide und Carbonate der Erdalkalimetalle, vorzugsweise des Calciums. Natriumhydroxid ist als basische Verbindung und damit als Neutralisationsmittel besonders bevorzugt.

Die basischen Verbindungen können in fester Form oder als alkoholische oder wäßrige Lösungen und in Mengenverhältnissen, die mindestens für die Neutralisation der Gesamtmenge der Sulfonsäuren ausreichen, eingesetzt werden. Vorzugsweise arbeitet man mit einer Menge, die etwas größer als die stöchiometrisch erforderliche Menge ist, z. B. bis zu 1,2 Val der basischen Verbindung pro Val Sulfonsäure.

Aus der so behandelten alkoholischen Lösung werden die sulfonsauren Salze sodann in bekannter Weise, z. B. über einen Dünnschichtverdampfer, von dem Extraktionsmittel, dem noch vorhandenen nicht abgetrennten Paraffin und dem Wasser befreit. Die Sulfonate werden dabei als Schmelze aus dem Sumpf des Verdampfers abgezogen. Als Kopfprodukt wird ein Gemisch, bestehend aus dem als Extraktionsmittel eingesetzten aromatischen Alkohol, dem restlichen Paraffin und Wasser erhalten, das sich unter Ausbildung von drei Phasen in die Bestandteile Alkohol, Paraffin und Wasser auftrennt, die nach entsprechender Aufarbeitung — ebenso wie das aus der 1. Trennoperation stammende Paraffin — in das Verfahren zurückgeführt werden können.

Das erfindungsgemäße Verfahren wird insbesondere auf die Behandlung von rohen Sulfonierungslösungen angewandt, die aus der Sulfoxidation von n-Paraffinen stammen, deren Moleküle 7 bis 30 und vorzugsweise 10 bis 20 Kohlenstoffatome aufweisen.

Die nach dem vorliegenden Verfahren gewonnenen bzw. isolierten Produkte, freie Sulfonsäuren bzw. deren Alkali- oder Erdalkalisalze, dienen vornehmlich zur Herstellung von Detergentienformulierungen.

Die nachfolgenden Beispiele dienen zur Erläuterung des erfindungsgemäßen Verfahrens.

Alle Prozentangaben sind, sofern nicht anders angegeben, Gewichtsprozent.

## Beispiel 1

6000 g eines Sulfoxidationsaustrags der Zusammensetzung

| | |
|---|---|
| Paraffinöl ($C_{14}-C_{17}$) | 35,6 Gewichtsprozent |
| Monosulfonsäure | 20,9 Gewichtsprozent |
| Disulfonsäure | 2,7 Gewichtsprozent |
| Schwefelsäure | 7,2 Gewichtsprozent |
| Wasser | 33,6 Gewichtsprozent |

werden mit 3000 g Benzylalkohol versetzt und bei Raumtemperatur gut durchmischt. Nach kurzem Stehen (1 bis 2 Minuten) bilden sich drei klare und sehr gut voneinander abtrennbare Phasen aus. Die obere Phase (1728 g) enthält das Paraffingemisch und ca. 1% Benzylalkohol, die mittlere Phase (5518 g) den Extrakt der Sulfonsäuren in Benzylalkohol und die untere Phase (1752 g) eine wäßrige 22,4%ige Schwefelsäure.

Daraus ergibt sich ein Abtrennungsgrad von 90,8% für die Schwefelsäure und von 80,1% für das Paraffin, jeweils bezogen auf die ursprünglich im Gemisch vorhandenen Mengen Schwefelsäure und Paraffinöl.

Die abgetrennte mittlere Phase wird anschließend mit 50gewichtsprozentiger wäßriger Natronlauge auf einen pH-Wert von 11 bis 12 eingestellt. Die Natriumsalze der Sulfonsäuren werden sodann durch Destillation im Vakuum (10 mbar) von dem Lösungsmittel, den restlichen Mengen Paraffin und dem Wasser befreit. Der erhaltene helle Rückstand läßt sich gut pulverisieren. Er hat folgende Zusammensetzung:

| | |
|---|---|
| Monosulfonat | 83,8 Gewichtsprozent |
| Di- und Polysulfonat | 7,9 Gewichtsprozent |
| Paraffin | 0,4 Gewichtsprozent |
| Natriumsulfat | 4,5 Gewichtsprozent |
| Natriumhydroxid | 0,3 Gewichtsprozent |

Beispiel 2

400 g eines Sulfoxidationsproduktes (Reaktoraustrag) der Zusammensetzung

| | |
|---|---|
| Paraffin $C_{14}-C_{17}$ | 33,9 Gewichtsprozent |
| Monosulfonsäure | 20,4 Gewichtsprozent |
| Disulfonsäure | 2,8 Gewichtsprozent |
| Schwefelsäure | 7,7 Gewichtsprozent |
| Wasser | 34,7 Gewichtsprozent |

werden mit 200 g $\beta$-Phenylethanol vermischt. Es bilden sich in kurzer Zeit 3 sehr gut voneinander trennbare Phasen aus:

| | |
|---|---|
| obere Phase | (104,7 g) |
| mittlere Phase | (386 g) |
| untere Phase | (126,1 g) |

Die untere Phase weist einen Gehalt von 22,2 Gewichtsprozent Schwefelsäure auf. Daraus errechnet sich ein Abscheidungsgrad von 91% für die Schwefelsäure. Die obere Paraffinphase enthält etwa 1 Gewichtsprozent Benzylalkohol, so daß sich, bezogen auf die im Reaktoraustrag vorhandene Paraffinmenge, ein Abscheidungsgrad von 76,4% für das Paraffin ergibt.

Beispiel 3

Dasselbe Ausgangsmaterial wie im Beispiel 2 wird mit $\alpha$-Phenylethanol (0,5 Gewichtsteile auf 1 Gewichtsteil Reaktoraustrag) versetzt. Die drei entstehenden Phasen werden getrennt. Man findet für das Paraffin eine Abscheidung von 55%, für die Schwefelsäure 96%, jeweils bezogen auf die ursprüngliche vorhandene Menge.

Beispiel 4

1 Gewichtsteil desselben Ausgangsmaterials wie im Beispiel 2 wird mit 0,5 Gewichtsteilen 3-Phenylpropanol-(1) versetzt, durchmischt und die entstehenden 3 Schichten getrennt. Es werden 93,7% der ursprünglich vorhandenen Schwefelsäure in der unteren Phase und 62,6% des Paraffins in der oberen Phase abgetrennt.

**Patentansprüche**

1. Verfahren zur Abtrennung von Sulfonsäuren aus dem bei der Umsetzung von Paraffinen mit Schwefeldioxid, Sauerstoff und Wasser in Gegenwart von ultraviolettem Licht erhaltenem Reaktionsprodukt mit Hilfe eines Alkohols als Extraktionsmittel, dadurch gekennzeichnet, daß man das Reaktionsprodukt mit einem aromatischen Alkohol der allgemeinen Formel

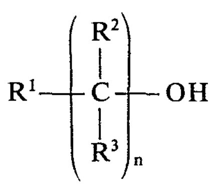

behandelt, in der $R^1$ einen gegebenenfalls durch Halogenatome und/oder Alkylgruppen mit 1 bis 6 Kohlenstoffatomen substituierten Phenylrest darstellt, $R^2$ und $R^3$ Wasserstoff bedeuten, n eine ganze Zahl von 1 bis 3 ist und $R^2$ auch eine Methylgruppe sein kann, wenn n gleich 1 ist, die alkoholische Phase von den übrigen Phasen abtrennt und daraus die Sulfonsäuren isoliert.

2. Verfahren nach Anspruch 1, dadurch gekennzeichnet, daß man als aromatischen Alkohol Benzylalkohol einsetzt.

3. Verfahren nach den Ansprüchen 1 und 2, dadurch gekennzeichnet, daß man den Sulfoxidationsaustrag mit 20 bis 100 Gewichtsprozent des aromatischen Alkohols behandelt.

## Claims

1. A process for the separation of a sulphonic acid from the reaction product obtained in the conversion of a paraffin with a sulphur dioxide, oxygen and water in the presence of ultraviolet light, using an alcohol as extraction agent, characterised in that the reaction product is treated with an aromatic alcohol of the general formula

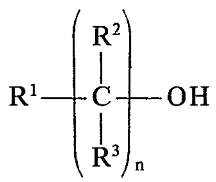

where $R^1$ is phenyl optionally substituted by halogen and/or alkyl of 1 to 6 carbon atoms, $R^2$ and $R^3$ are hydrogen and n is an integer from 1 to 3, provided that $R^2$ can also be methyl when n is 1, the alcoholic phase is separated fromt the other phases and the sulphonic acid is isolated from it.

2. A process according to claim 1, characterised in that benzyl alcohol is used as the aromatic alcohol.

3. A process according to claim 1 or 2, characterised in that the sulphoxidation product mixture is treated with 20 to 100 percent by weight of the aromatic alcohol.

## Revendications

1. Procédé de séparation d'acides sulfoniques à l'aide d'un alcool comme agent d'extraction, à partir du produit de réaction obtenu en faisant réagir des paraffines sur du dioxyde de soufre, de l'oxygène et de l'eau en présence de lumière ultraviolette, caractérisé par le fait qu'on traite le produit de réaction par un alcool aromatique de la formule générale

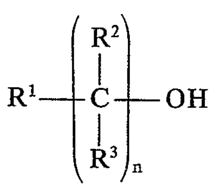

dans laquelle $R^1$ représente un reste phényle éventuellement substitué par des atomes d'halogène et/ou par des groupes alkyle comportant de 1 à 6 atomes de carbone, $R^2$ et $R^3$ représentent de l'hydrogène, n est un nombre entier d'une valeur de 1 à 3 et $R^2$ peut également être un groupe méthyle lorsque n est égal à 1, qu'on sépare la phase alcoolique des autres phases et qu'on en isole les acides sulfoniques.

2. Procédé selon la revendication 1, caractérisé par le fait qu' on utilise l'alcool benzylique comme alcool aromatique.

3. Procédé selon les revendications 1 et 2, caractérisé par le fait qu'on traite le produit provenant de la sulfoxydation par 20 à 100% en poids de l'alcool aromatique.